# EUROPEAN PATENT SPECIFICATION

(11) **EP 1 803 732 B1**
(45) Date of publication and mention of the grant of the patent: **22.02.2012**
(21) Application number: 05794474.6
(22) Date of filing: 22.09.2005
(51) Int. Cl.: C07K 14/47, A61K 38/17

(54) **PEPTIDES AND APL-TYPE DERIVATIVES OF HSP60 AND PHARMACEUTICAL COMPOSITIONS**
PEPTIDE UND DERIVATE VOM APL-TYP VON HSP60 UND PHARMAZEUTISCHE ZUSAMMENSETZUNGEN
PEPTIDES ET DERIVES DU TYPE APL DE LA HSP60 ET COMPOSITIONS PHARMACEUTIQUES

(30) Priority: 24.09.2004 CU 2072004
(43) Date of publication of application: 04.07.2007
(60) Divisional application: 10189769.2
(73) Proprietor: CENTRO DE INGENIERIA GENETICA Y BIOTECNOLOGIA (CIGB), Ciudad de la Habana 10600 (CU)
(72) Inventor: DOMÍNGUEZ HORTA, María del Carmen, Calle: 186, Ciudad de La Habana 10 600 (CU); PADRÓN PALOMARES, Gabriel R., Avenida 31, Ciudadde La Habana 10 600 (CU); LÓPEZ MARÍN, Nelia, Avenida 31, Ciudad de La Habana 10 600 (CU); LORENZO PEREZ, Norailys, Calle: 26, Ciudad de La Habana 14 000 (CU); BARBERÁ BETANCOURT, Ariana, Calle: 403, Ciudadde La Habana 17 200 (CU); HERNÁNDEZ GARCÍA, Ariadna, Calle: 26, Ciudad de La Habana 10 600 (CU); MORERA CORDOVA, Vivian, Calle: Lagueruela Oeste, 10de Octubre, Ciudad de La Habana 10 500 (CU); COSME DÍAZ, Carelia, Avenida 31, Ciudad de La Habana 10 600 (CU); MERINO GARCÍA, Nelson J., Avenida: 230, Ciudad deLa Habana 13 600 (CU); VÁZQUEZ BONACHEA, Ariel, Calle: 186, Ciudad de La Habana 10 600 (CU); SUÁREZ ALBA, José, Calle: Playita, Ciudad de La Habana 10 700 (CU)
(74) Representative: van Loon, C.J.J.
(86) International application number: PCT/CU2005/000008
(87) International publication number: WO 2006/032216

(56) References cited:
- WO-A-01/43691
- WO-A-97/11966
- RAMMENSEE HANS-GEORG ET AL: "SYFPEITHI: Database for MHC ligands and peptide motifs" IMMUNOGENETICS, SPRINGER VERLAG, BERLIN, DE, vol. 50, no. 3-4, November 1999 (1999-11), pages 213-219, XP002183663 ISSN: 0093-7711 cited in the application
- SINGH H ET AL: "ProPred: prediction of HLA-DR binding sites." BIOINFORMATICS (OXFORD, ENGLAND) DEC 2001, vol. 17, no. 12, December 2001 (2001-12), pages 1236-1237, XP002371461 ISSN: 1367-4803 cited in the application
- QUINTANA FRANCISCO J ET AL: "DNA fragments of the human 60-kDa heat shock protein (HSP60) vaccinate against adjuvant arthritis: identification of a regulatory HSP60 peptide." JOURNAL OF IMMUNOLOGY (BALTIMORE, MD. : 1950) 1 OCT 2003, vol. 171, no. 7, 1 October 2003 (2003-10-01), pages 3533-3541, XP002371462 ISSN: 0022-1767
- PRAKKEN BERENT J ET AL: "Epitope-specific immunotherapy induces immune deviation of proinflammatory T cells in rheumatoid arthritis." PROCEEDINGS OF THE NATIONAL ACADEMY OF SCIENCES OF THE UNITED STATES OF AMERICA. 23 MAR 2004, vol. 101, no. 12, 23 March 2004 (2004-03-23), pages 4228-4233, XP002371463 ISSN: 0027-8424
- KAMPHUIS S S ET AL: "V1.2. recognition of multiple hsp60 epitopes in patients with juvenile idiopathic arthritis opens the way for antigen-specific immunotherapy." ANNALS OF THE RHEUMATIC DISEASES. OCT 2001, vol. 60 Suppl 2, October 2001 (2001-10), pages II7-II15, XP002371476 ISSN: 0003-4967
- CHOI J-I ET AL: "Epitope mapping of Porphyromonas gingivalis heat-shock protein and human heat-shock protein in human atherosclerosis." JOURNAL OF DENTAL RESEARCH. DEC 2004, vol. 83, no. 12, December 2004 (2004-12), pages 936-940, XP002371464 ISSN: 0022-0345
- KAMPHUIS SYLVIA ET AL: "Tolerogenic immune responses to novel T-cell epitopes from heat-shock protein 60 in juvenile idiopathic arthritis." LANCET. 2005 JUL 2-8, vol. 366, no. 9479, 2 July 2005 (2005-07-02), pages 50-56, XP002371481 ISSN: 1474-547X
- JOOSTEN I ET AL: "DIRECT BINDING OF AUTOIMMUNE DISEASE RELATED T CELL EPITOPES TO PURIFIED LEWIS RAT MHC CLASS II MOLECULES" 1 May 1994 (1994-05-01), INTERNATIONAL IMMUNOLOGY, OXFORD UNIVERSITY PRESS, GB, PAGE(S) 751-759 , XP000590812 ISSN: 0953-8178 page 757, left-hand column, paragraph 2; table 2
- REIZIS B ET AL: "The peptide binding specificity of the MHC class II I-A molecule of the Lewis rat, RT1.BI." INTERNATIONAL IMMUNOLOGY DEC 1996, vol. 8, no. 12, December 1996 (1996-12), pages 1825-1832, XP002421975 ISSN: 0953-8178

## Description

### Field of the invention

The present invention is related with peptides of human heat shock protein of 60 kDa (abbreviated *hHsp60),* and Altered Peptide Ligands (abbreviated APL) derived from them. Also, the invention is related with the pharmaceutical compositions comprising such peptides for the treatment of Rheumatoid Arthritis (RA).

### Background art

The RA is an autoimmune disease of unknown etiology that affects approximately 1% of world's population. It is a syndrome characterized by chronic inflammation of the joints, although systemic manifestations can be also observed. This illness begins with the inflammation of the synovial membrane and frequently causes the erosive destruction of the adjacent cartilage and the bone, which result in moderate physical inability of 80% of the patients and an early mortality (Moctezuma, J.F. (2002) Manifestaciones articulares de la Artritis Reumatide. Revista Mexicana de Reumatología 17:211-219). RA can be presented at any age, without distinctions of races or ethnic groups, but the maximum incidence of its beginning happens between 25 and 55 years old. Among people with RA, the women overcome the men in a proportion from three to one (Emery, P. (2002) Early referral recommendation for newly diagnosed rheumatoid arthritis: evidence based development of to clinical guide. Ann Rheum Dis. 61:290-297).

The cause of RA is unknown. It is an illness that involves the presence of genetic, environmental, immunological and hormonal factors. Certain genes have a role in the immune system, associated with a tendency to develop RA. At the same time, some people with RA don't have these genes and other people having them never develop the illness. Therefore, it has been suggested that the genetic background is important but it is not decisive.

In models of autoimmune disease, microbial antigens with similar structure to own antigens can release a crossed response to autoantigens, producing an alteration in the mechanisms of tolerance and perpetuating an autoimmune response. In general sense, the damage and local necrosis in a tissue produced by an infectious agent could discover the autoantigen cryptic epitope, being able to activate autoreactivate T cells (Albert, L.J. (1999) Mechanisms of Disease: Molecular Mimicry and autoimmunity. N Engl J Med 341:2068-2074)

The phase of transition of T lymphocyte between tolerance and immunity/ autoimmunity is regulated at different levels. Two important parameters in this transition are the state of maturation of the Antigen Presenting Cells (abbreviated APC) and the levels of autoantigens that are detected by the immune system (Ohashi, P.S. (2002) Making and breaking tolerance. Current Opinion in Immmunology 14:744-759).

One of the current hypotheses that tries to explain the development of autoimmune diseases, outlines that APC in absence of signs of the innate immune system or of signs of danger, remain relatively immature and induce tolerance in autoreactive T cells, when own peptides are presented to them (Steiman, R.M. (2000) The induction of tolerance by dendritic cells that have captured apoptotic cells. J Exp Med. 191:411-416). The induction of peripheral tolerance is also correlated with the concentration of own antigen (Kurts, C. (1999) CD8 T cell ignorance or tolerance to islet antigens depends on antigen dose. PNAS 96:12703-12707). An increment in the presentation of own antigens due to the increment of their expression levels, allows their detection by autoreactive ignorant T cells. If levels of own antigens are increased in absence of events that promote the maturation of APC, the tolerance to these antigens is maintained, otherwise, it happens in presence of proinflammatory signs or other events that promote the maturation of the APC, the tolerance is breaks by activation of the ignorant T cells and autoimmune diseases are developed (Janeway, C.A. (2002) Innate immune recognition. Annu Rev Immunol. 20:197-216). The infectious agents that have been object of study like cause of RA are: Epstein Bar virus, the retrovirus, virus of the hepatitis C, the *Mycobacterium tuberculosis* (abbreviated *Mt*) and the *Helicobacter pylori,* among others.

The pathogenesis of RA is characterized by the concerted action of different types of cells that cause the progressive destruction of the cartilage and the bone.

In normal situations a balance exists among the inflammatory cytokine as: TNFα, IL-1, IL-6, IL-15, IL-16, IL-17, IL-18 and the IFNγ, and the anti-inflammatory ones as IL-4, IL-11, IL-13 and antagonistic of IL-1 or TNFα. In the RA this balance moves however in favor of the inflammatory cytokines (Arend, W.P. (2001) Cytokine imbalance in the pathogenesis of rheumatoid arthritis: The role of interleukin-1 receiving antagonist. Semin Arthritis Rheum 30(2):1-6)

The recognition of an exogenous antigen or autoantigen is probably the reason of a series of events that cause destruction of the joints in patients with RA. This phenomenon cause the activation of T CD4+ lymphocytes that in cooperation with the stimulation of different cytokines, induces their differentiation to cells Th1 with the consequent liberation of proinflammatory cytokines (IL-2 and INFγ) (Simón, A.J. (2001) Biological therapy in Rheumatoid Arthritis. Magazine of Clinical Investigation 53(5):452-459). Many investigators coincide that the chronic inflammation of the joints is induced by these activated T cells that infiltrate the synovial membrane. The action of these cytokines on macrophages causes the production of high amount of TNFα and IL-1. These cause a series of local and systemic actions as regulating the expression of the molecules of adhesion in the endothelial cells (LFA1, ICAM-1), which recruit other cells to the inflammation sites. They also stimulate macrophages, fibroblasts, condrocytes and osteoclasts to the liberation of others mediators of the inflammation, as IL-15 and IL-8. TNFα and IL-1 stimulate the proliferation of the synovial membrane that causes formation of pannus, they can also induce the differentiation of B lymphocytes to cells producing antibodies that potentially participate in the destruction of the joint. They also inhibit the production of anti-inflammatory cytokines (IL-4 and IL-14) produced by Th2 cells and stimulate hepatocytes to liberate IL-6. IL-6 favors the production of the proteins of the acute phase, which participate strengthening the immune response (Forre, O. (2000) New possibilities of treatment in AR. Scand J Rheumatol 29(2):73-84).

Among autoantigens involved in the pathogenesis of RA is *Hsp60,* a protein that belongs to the family of the *Hsp,* which are immunogenic proteins with exceptionally evolutionary conservation. The immune response against the strange *Hsp* is an important mechanism of defense against bacterial infections. The antibodies against these proteins can be abundant in healthy people and in patient with autoimmune illnesses and they can cross react with the own antigens (Chen, W. (1999) Human 60-kDa Heat-Shock Protein: To Danger Signal to the Innate Immune System. J Immunol. 162:3212-3219).

The *Hsp65* of *Mt* is homologous to the *Hsp60* of the mammals. This suggests that the *Hsp60* can be recognized as autoantigen in patient with RA. Comparing patients with osteoarthritis and patients with RA, these last ones have an increase of prolipherative response of B lymphocytes in the synovial liquid to the *Hsp65* of mycobacterium. The intensity of the response correlates with the synovial inflammation. This response is not specific for RA compared with other inflammatory illnesses (Life, P. (1993) Responses to Gram negative enteric bacterial antigens by synovial T cells from patiens with juvenile chronic arthritis: recognition of heat shock protein hsp60. J Rheumatol. 20:1388-1396).

The concentration of Hsp is a possible sign of danger for the immune system, which are released from dead cells, and can induce an inflammatory response and to begin the maturation of the APC. The *Hsp*, are intracellular proteins, which are not expressed in the cellular membrane, neither are secreted, so that the *Hsp* are attractive candidates for molecules that constitute signs of danger (Van den Berg, WB. (1998): Joint inflammation and cartilage destruction may occur uncoupled. Springer Semin lmmunopathol. 20:149-164).

Several preparations have been proposed using the *Hsp60* or its derived peptides for the treatment of some autoimmune pathologies. For example in the patent EPO262710, the use of several peptides of the *Hsp60* of *Mt* is proposed for the treatment and diagnosis of autoimmune illnesses, especially of arthritic conditions. This invention is based on the fact that previous infections with several bacteria can trigger the development of autoimmune illnesses, in genetically susceptible people, for example: patients with RA can show a high reactivity to microbial antigens. These same inventors in the patent EPO322990 propose the use of others peptides of the *Hsp60* of *Mt* with the same purpose of the previous patent.

In the patent application W09610039 they intend the use of a peptide of the *Hsp60* of *Mt* for the diagnosis and treatment of autoimmune arthritis.

In the patent application WO9711966, the authors intend the use of peptides of non conserved regions of the *hHsp60,* that don't coincide with the *Hsp60* of bacteria that could induce tolerance in T cells of patients with RA.

The patent application WO0143691 proposes the use of pharmaceutical preparations composed specifically by peptides of the *hHsp60* and their variants for the prevention of inflammatory illnesses such as RA.

In the patent US6180103 the authors intend the use of a peptide of the *Hsp60* called p277 and its analogous for the diagnosis and prevention of the diabetes type I.

The patent US5993803 protects the use of the *Hsp60,* the peptide p277 and a group of derived peptides of this protein to reduce the severity of the immune response during the transplant of organs.

Quintana et al (J Immunol 2003, 171: 3533-3541) identified a HSP60-derived peptide consisting of residues 31- 50. Vaccination with this peptide inhibited the development of Adjuvant Arthritis in rats. The peptide was not tested for its regulatory properties in patients with RA.

Recently it has been considered that the atherosclerosis presents a series of similar characteristics to autoimmune processes. In the patent application WO0072023 the authors purpose a method for the treatment and diagnosis of the atherosclerosis and coronary illnesses using a preparation that contains the protein *Hsp60.*

In the patent application WO02057795, a new method is protected for the diagnosis and treatment of the osteoporosis using proteins of the family of *Hsp* and proteins from pathogens such as: viruses and bacteria.

At the present time, cure doesn't exist for the RA. The current methods of treatment are centered in alleviating the pain, to reduce the inflammation, to retard the damages of the joints and to improve the functions and the well-being of the patients. Recently immunomodulating medications have been elaborated, that blocks cytokines that participate in the beginning and maintenance of the inflammatory response in RA, with the purpose to stop or slow the progression of the illness. For this kind of therapy two types of medications exist: blocking the action of the Tumor Necrosis Factor (abbreviated TNFα) and those that inhibit the action of the interleukin 1 (IL-1).

Although the results are promissory with the therapies anti-TNFα and anti-IL-1, the percentage of infections is high. Many of treated patients with these drugs develop serious infections that are fatal in some cases; including other autoimmune illnesses, neoplasias, etc. Besides, they are very expensive medications (Breshinan, B. (1998) Treatment of rheumatoid arthritis with recombinant human anti-interleukin-1 antagonist. Arthritis Reum. 41:2196-2204)

Oral tolerance has been proposed as a method of creating peripheral tolerance in front of certain antigens. This can be induced by mechanisms of active suppression, anergy or clonal deletion, depending on the doses and the frequency of the administration of the antigen. The method can induce regulatory T cells that are activated in a specific way by the antigen, but exert its action independently (active suppression). For the regulatory action takes place, it is not necessary to administer the supposedly pathogenic antigen, but any other able to induce the active suppression in the inflammatory focus, inhibiting the activity of the pathogenic effector cells. Collagen type II (abbreviated CII) is the autoantigen that has been used more frequently in this sense. The results of the studies carried out in patients with RA, using chicken and bovine CII have given contradictory results (Trentham, D.E. (1993) Effects of oral administration of type II collagen in rheumatoid arthritis. Science 261:1727-1730; Sieper, J. (1996) Oral type II collagen treatment in early rheumatoid arthritis: to double-blind, placebo-controlled, randomize trial. Arthritis Rheum. 39:41-51).

The patent US6153200 intends the use of a peptide of the *Hsp70,* protein belonging to the family of *Hsp,* to induce tolerance for oral route in patients with RA.

Another variant that has been suggested to induce tolerance is through APL peptides, based on the fact that T cells are activated if the specific T CD4+ lymphocytes for a certain antigenic peptide recognize the antigen presented by competent APC. Nevertheless, if the same T cell is first activated with a different form from the antigen, in which one of the contact sites with the TcR are lightly altered, it can result in a partial activation or even inactivation of the T cells. These antigens are named APLs. The APLs are similar to immunogenic peptides with one or several substitutions in the essential positions of contact with the TcR or with MHC that interfere the cascade of necessary events for the complete activation of the T cells.

Conceptually, APLs can be designed with similar properties to the immunogenic peptide (agonist) among other effects for increasing the response of T cells toward specific antigens. This effect is advantageous under pathological conditions as infectious illnesses. Peptides can also be designed with antagonistic properties to the immunogenic peptide that could be beneficial in the control of autoimmune illnesses since they can block the response of T cells acting as antagonistic of the TcR (M. De Magistris (1992) Antigen analog-major complex histocompatibility complexes act as antagonist of the T cell receptor. Cell 68:625 - 634), partial agonist or inducing a population of regulatory T cells that mediate the active suppression (Evavold, B.D. (1991) Separation of IL-4 production from Th cell proliferation by an altered T cell ligand. Science 252:1308-1310).The capacity to experimentally manipulate the intrinsic properties of peptide ligands allows altering the nature, the course and the power of the immune cellular response appropriately.

Up to this moment, two clinical trials in humans using type APL peptides have been performed for the treatment of autoimmune diseases. In both assays, the peptides are derived from an epitope in position 83 to 93 of the myelin basic protein. One of these trials included 142 patients with multiple sclerosis, and it was suspended because 9% of the patients developed hypersensitivity (Ludwig Kapposi (2000) Induction of non-encephalitogenic type 2 T helper-cell autoimmune response in multiple sclerosis after administration of an altered peptide ligand in to placebo controlled, randomized phase II trial. Nature Medicine 10:1176-1182).

The other trial included 25 patients and was also interrupted, because in three patients was observed an exacerbation of the illness (Bibiana Bielekova (2000) Encephalitogenic potential of the myelin basic protein peptide (amino acids 83-99) in multiple sclerosis: Results of to phase II clinical trial with an altered peptide ligand. Nature Medicine 6:1167-1175). The main author of this work analyzes the possible factors that determined these negative results, and outlines that the changes carried out in the APL's sites may originate new motifs of union to the HLA (like it happened in the case of the APL binding to DRB1*0404 present in patient I), the complex APL-HLA can also stimulate T cells not eliminated in the negative selection that can be cross-activated by the native autoantigen. In this trial high concentrations of the APL were used in the pharmaceutical preparations, which can stimulate T cells with high similarity for the autoantigen and to induce a heteroclitic T cells response. (Bibiana Bielekova and Roland Martin (2001) Antigen-specific immunomodulation via altered peptide tying. J Mol Med. 79:552-556).

For carrying out these two clinical trials, the authors didn't previously analyze the *in vitro* response of the T cells from the patients to the APL. They also did not consider the types of HLA molecules expressed by the treated patients

The main challenge in the treatment of the autoimmune diseases is the development of therapeutic strategies that can eliminate pathogenic T cells with specificity, without affecting other non related T cells. Therefore, therapeutic search should find a secure, specific and effective way of turning off an advanced autoimmune process.

The present invention, in contrast to the state of the previous technique, proposes the use of peptides of the *hHsp60* and its derived type APL, which induce inhibitory molecular mechanisms, in a specific way, of the course of RA.

### Summary of the invention

The present invention solves the problem previously mentioned, providing peptides of the human protein of heat shock of 60 kDa that constitute epitopes for T cells, to induce mechanisms of tolerance, in particular anergy induction mechanisms or mediated by clones of regulatory T cells in patients with RA Peptides tested in respect thereto are represented by the sequences:
- E18-12: MGPKGRTVIIEQSWGSPKVTK (SEQ. ID. NO: 1)
- E18-3: SIDLKDKYKNIGAKLVQOVANNTNEEAG (SEQ. ID. NO: 2)
- F19-6: IIDPTKVVRTALLDAA (SEQ. ID. NO: 3)

The patent application WO9711966, intends the use of peptides of the *hHsp60* and of *Mt* based on the fact that they could induce tolerance in T cells and to prevent the RA. These authors corroborate certain improvement of the illness by administering these peptides to mice (Arthritis was previously induced with pristine). Contrary to this application, we have demonstrated that our peptides induce mechanisms of active suppression which induce peripheral tolerance in a very efficient way, corroborating a significant increase of IL-10 in two animal models of Arthritis, as well as in "ex vivo" assays using mononuclear cells of patients.

It is well known that fundamental response of T cells in the model of adjuvant-induced arthritis (AIA) is directed against the *Hsp60.* We checked in this model that our derived peptides of the *hHsp60* exert a very marked therapeutic protection, producing an increase of the IL-10 and a decrease of TNFα levels. The same therapeutic effect exerted by these peptides, we have also checked in the animal model of collagen- induced arthritis (CIA), where the major response is against the type II collagen.

These facts indicate that therapeutic capacities of our peptides are independent of the induction agent and can mediate mechanisms of active suppression in the site of inflammation that could be extended to other autoantigens present in joints, which progressively contribute to the inmunopathogenic process that takes place during the course of RA. These results reinforce the therapeutic possibilities of the use of our preparations for the treatment of RA.

The peptides which are particular useful, according to the present invention, are the derived type APL variants of the peptide E18-3 (SEQ. ID. NO: 2), modified in the contact sites with the human MHC molecule. The amino acid sequences of these APLs are with a substitution in position 1 for: L,I,V,M,Y,W,F, or A
position 2 for: A,D,E,F,G,H,I,K,L,M,N,Q,R,S,T,V,W, or Y
position 3 for: A,F,I,K,L,M,P,R,S,T,V,W, or Y
position 4.for: L,I,V,M,Y,W,F, or A
position 5 for: A,D,E,F,G,H,I,K,L,M,N,Q,R,S,T,V,W, or Y
position 5 for: A,D,E,F,G,H,I,K,L,M,N,Q,R,S,T,V,W, or Y
position 7 for A,F,I,K,L,M,P,R,S,T,V,W, or Y
position 8 for A,D,E,F,G,H,I,K,L,M,N,Q,R,S,T,V,W, or Y

In a particular carrying out, derived type APL peptides have amino acid sequence selected from the group that consists of SEQ. ID. NO: 14, SEQ. ID. NO: 15, SEQ. ID. NO: 16, SEQ. ID. NO: 17, SEQ. ID. NO: 18, SEQ. ID. NO: 19, SEQ. ID. NO: 20 and SEQ. ID. NO: 21.

The peptides of the present invention can be produced by routine methods of peptide synthesis. The specific composition of each peptide can be assayed by the level and the quality of the immune response that it induces in experiments as those are described in the examples that will be presented later on.

All the sequences described above and in the examples are useful and can also be used as a base for the design and synthesis of derived peptides with improved properties.

The invention also includes pharmaceutical compositions that include one or more of previously enumerated peptides and optionally a pharmaceutically acceptable vehicle.

The administration of the pharmaceutical preparations to the patients will be in correspondence with the HLA class II molecules that are expressed by the specific patient.

The way of administration of the pharmaceutical compositions will depend on the cytokines pattern that these preparations induce in the "ex vivo" tests. The pharmaceutical preparations containing APL peptides inducing a regulatory response will be administered by intradermal or subcutaneous route. The pharmaceutical preparations containing the original peptides inducing a TH1 response will be able to be administered by oral route.

The amounts of peptides in the pharmaceutical compositions of this invention are those that produce an effective immune response in the host. The effective amount is the quantity that when is administered induces molecular mechanisms that significantly diminish the inflammatory signs characteristic of the RA and stop the damages in the joints characteristic of the course of this illness. The amount of the pharmaceutical composition administered to the host can also vary depending on of several factors that include: the used peptides, ACR score (American College of Rheumatology, abbreviated ACR), HLA type II, time of having diagnosed the illness, age, sex, general health, as well as the level of immunological response in general.

The present invention also relates a method of treatment of the RA, which includes the administration to a patient of effective amounts of peptides or the pharmaceutical compositions referred previously.

### Brief description of the drawings

In part, results are shown graphically in the following figures:
**Figure 1****:** Evaluation of clinical signs of the Arthritis in rats treated with F19-6 and F19-7 peptides by intradermal route, in AIA model.
**Figure 2****:** Modulation of the levels of TNFα induced by F19-6 and F19-7 peptides in mononuclear cells of rats in the AIA model at day 15 after the induction of disease.
**Figure 3****:** Modulation of the levels of IL-10 induced by F19-6 and F19-7 peptides in mononuclear cells of rats in the AIA model at day 15 after the induction of disease.
**Figure 4****:** Optical microscopy of a section of the joint belonging to a sick not treated animal. IS: intra-articular space, EC: erosion of the cartilage, P: pannus. Stained with hematoxiline-eosine.
**Figure 5****:** Optical microscopy of a section of a joint belonging to an animal treated with F19-7 peptide by intradermal route. IS: intra-articular space. Stained with hematoxiline-eosine.
**Figure 6****:** Modulation of levels of TNFα induced by F19-6 and F19-7 peptides in mononuclear cells of rats in the CIA model at day 21 after the induction of disease.
**Figure 7****:** Modulation of levels of IL-10 induced by F19-6 and F19-7 peptides in mononuclear cells of rats in the CIA model at day 21 after the induction of disease.
**Figure 8****:** Modulation of the levels of TNFα by F19-6 and F19-7 peptides in mononuclear cells of patients with RA.
**Figure 9****:** Modulation of the levels of TNFα by E18-3 and E18-12 peptides in mononuclear cells of patients with RA.
**Figure 10****:** Modulation of the levels of IL-10 by E18-3 and E18-12 peptides in mononuclear cells of patients with RA.
**Figure 11****:** Modulation of the levels of IL-10 induced by E18-3 and E18-12 peptides and their APLs in mononuclear cells of patients with RA.
**Figure 12****:** Modulation of TNFα levels induced by E18-3 and E18-12 peptides and their APLs in mononuclear cells of patients with RA.
**Figura 13:** Ratio of IL-10 and TNFα levels, induced by E18-3 and E18-12 peptides and their APLs.
**Figure 14****:** Evaluation of clinical signs of Arthritis in rats treated with E18-12 and E18-12APL1 peptides by intradermal route, in AIA model.

### Detailed description /Examples

### Example 1: Peptides of the hHsp60 presented by human HLA.

The selected peptides of the *hHsp60* were those reported as epitopes of human T cells by the used programs: SYFPEITHI (Hans-Georg Rammensee, Jutta Bachmann, Niels Nikolaus Emmerich, Oskar Alexander Bachor and Stefan Stevanovic. 1999. Immunogenetics 50:213-219. SYFPEITHI: database for MHC ligands and peptide motifs. (access via: http://www.uni-tuebingen.de/uni/kxi /)) and ProPred (Singh,H. and Raghava,G.P.S. 2001. ProPred: Prediction of HLA-DR binding sites. Bioinformatics, 17(12):1236-37).

The sequences corresponding to these peptides are shown in Table 1.

**Table 1. Sequence of hHsp60 peptides presented by human HLA.**

| **Peptides** | **Position in the sequence** | **Amino acid Sequence** |
|---|---|---|
| E18-12 | 55-75 | MGPKGRTVIIEQSWGSPKVTK |
| | | (SEQ. ID. NO: 1) |
| E18-3 | 83-110 | SIDLKDKYKNIGAKLVQDVANNTNEEAG |
| | | (SEQ. ID. NO: 2) |

### Example 2: Peptide of the hHsp60 for epitope of Lewis rats.

Starting from the sequence of *hHsp60* we select a peptide that binds to the molecule of MHC-II of rat RT1.B1 according to the sequence motif described in the database SYFPEITHI (Hans-Georg Rammensee, Jutta Bachmann, Niels Nikolaus Emmerich, Oskar Alexander Bachor, Stefan Stevanovic. 1999. Immunogenetics 50:213-219. SYFPEITHI: database for MHC ligands and peptide motifs. (access via: http://www.uni-tuebingen.de/uni/kxi /).

A program that analyzes all peptides of 9 amino acids was designed to search those that fulfill with the motif. We also included variants, which in one of the positions were not included the described residuals as anchorage or preferentials.

The sequence corresponding to one of these peptides (F19-6) it is shown in the Table 2.

**Tabla 2. Sequence of hHsp60 peptide selected for rats.**

| **Peptide** | **Position in the sequence** | **Amino acid Sequence** |
|---|---|---|
| F 19-6 | 518-533 | IIDPTKVVRTALLDAA |
| | | (SEQ. ID. NO: 3) |

### Example 3: Design of APL with modifications in the amino acids involved in the binding to the MHC.

Starting from the peptides selected in examples 1 and 2, we have modified the positions involved in the binding to the specific MHC (positions 1,3,4,6,8 or 9) trying to increase the affinity of the peptide for the HLA molecule. It was searched for the optimal residuals in each one of these positions that could be anchorage residuals if the peptide was selected for methods that use binding motifs (SYFPEITHI) or residuals able to increase the score of the peptide as MHC ligand if the selection of the peptide was made using another algorithm type (ProPred).

As a result of these analyses, the peptide E18-12 has been substituted in the positions 1 to 7 by each one of the amino acids that are specified as follow: it is substituted in the position 1 for: A,F,I,L,M,V,W, or Y
position 2 for: A,D,E,F,G,H,I,K,L,M,N,Q,R,S,T,V,W, or Y
position 3 for: A,K,V,R,T,I,P,L,N,S,G,Y, or M
position 4 for: A,D,E,F,G,H,I,K,L,M,N,Q,R,S,T,V,W, or Y
position 5 for: A,D,E,F,G,H,I,K,L,M,N,Q,R,S,T,V, or Y
position 6 for: A,D,E,F,G,H,I,K,L,M,N,P,Q,R,S,T,V,W, or Y
position 7 for: A,D,E,F,G,H,I,K,L,M,N,Q,R,S,T,V, or Y it is substituted in the position 1 for L,I,V,M,Y,W,F, or A
position 2 for A,D,E,F,G,H,I,K,L,M,N,Q,R,S,T,V,W, or Y
position 3 for A,F,I,K,L,M,P,R,S,T,V,W, or Y
position 4 for L,I,V,M,Y,W,F, or A
position 5 for A,D,E,F,G,H,I,K,L,M,N,Q,R,S,T,V,W, or Y
position 6 for A,D,E,F,G,H,I,K,L,M,N,Q,R,S,T,V,W, or Y
position 7 for A,F,I,K,L,M,P,R,S,T,V,W, or Y
position 8 for A,D,E,F,G,H,I,K,L,M,N,Q,R,S,T,V,W, or Y

Peptide F19-6 has been substituted in the positions 1 and 2 by the amino acids that are specified as follow: position 1 is substituted by H.
position 2 is substituted by E.

### Example 4: Induction of Arthritis by adjuvant and by collagen in Lewis rats.

Female inbred Lewis rats (RT1.BI MHC) were used in the experiment supplied by the National Center for the Production of Laboratory Animals (CENPALAB, Cuba). The rats were randomly selected, with an approximate weight of 101-120 g and 5-8 weeks of age.

One of the inductor agents of the illness used was the *Mt* (H37Ra, Difco, England) in Incomplete Freund's Adjuvant (IFA) (Sigma, USA). The other inductor agent of the Arthritis was the bovine type II collagen in IFA.

In this example, two different induction agents were used for developing arthritis in rats by an immune response against two different autoantigens. It is very well known that in the animal model for Arthritis using *Mt* as inductor of the disease, the fundamental response of the T-cells is against the *Hsp60* (Anderton, SM (1995) Activation of T cells recognizing self 60-kD heat shock protein can protect against experimental arthritis. J. Exp. Med. 181: 943-952). In the model using collagen as inductor, noticeable antibody response as well as T-cell clones are developed against this antigen (M Griffiths (1988) Immunogenetics of collagen-induced arthritis in rats. Inten. Rev. Immunology 4: 1-15).

Our fundamental objective for developing these two Animal Models was to evaluate our peptides in both models, with the purpose of demonstrating if the protective effect of these peptides was independent or not to the autoantigen used as induction agent of the Arthritis in rats.

### Example 5: Evaluation of the clinical signs.

The inflammation grade associated with the development of the arthritis that presented each rat was evaluated according with the following average prepared for this purpose:
0 point: normal paw.
1 point: Slight, but definitive redness of the ankle or doll, apparent redness and inflammation limited to an individual finger, independently of the number of affected fingers.
2 points: Moderated redness and inflammation of the ankle and the doll.
3 points: Severe redness and inflammation of the whole paw including the fingers.
4 points: Maximum inflammation and deformity of the paw involving multiple articulations.

The four paws were evaluated separately according to the established score, each paw could receive up to 4 points. Therefore, each rat could receive a maximum of 16 points.

The visual evaluation was carried out after the induction of the illness every five days.

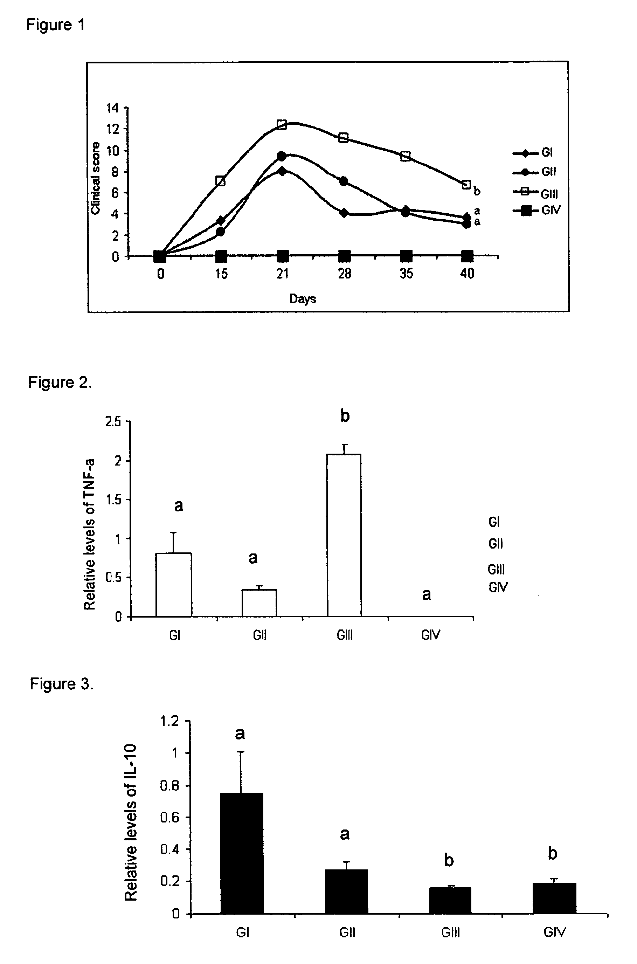

The clinical evaluation of the signs of the Arthritis demonstrated that there were significant statistical differences among animals corresponding to the healthy control group (group IV), where the illness was not induced, and animals corresponding to the sick control group (group III), where illness was induced but they were not treated with peptides, like it is shown in the Figure 1.

### Example 6: Peptides synthesis.

The peptides were synthesized by the strategy Fmoc/tBu in syringes. The resin used was Fmoc-AM-MBHA (0,54 mmol/g) and the synthesis protocol was continued with mechanical agitation. After the treatment with TFA, the peptide was lyophilized and it was characterized by HPLC and mass spectrometry.

### Example 7: Intradermal administration of peptides.

The animals were separated in four groups of 12 rats each one. The illness was induced in three groups of animals (Group I - Group III), and two groups were treated with peptides and one was the control of the induction of the illness. The two groups of animals that were treated with peptides received 200 µg of peptide per each rat in a volume of 100 µL of PBS, the days 12, 17 and 22 after the induction of the disease with *Mt.*

The animals separated in four groups were treated in the following way:
- group I: animals treated with peptide F19-6
- group II: animals treated with peptide F19-7
- group III: animals not treated with peptides (control of the induction of the illness)
- group IV: animals that were not induced with the illness and were not treated with peptides (negative control)

Days 15, 21, 28 and 35 after the induction, three animals were sacrificed by group, spleens were extracted and sample was taken from joints for histopathological analysis

As it is shown in the Figure 1 the signs associated with the development of the Arthritis began gradually. In the animals of group III, corresponding to the control group of the development of the illness, from day 10 after the induction of the Arthritis, it became evident the development of an inflammatory process that began with a slight redness of the inferior joints and that went expanding to the rest of the joints until became severe in some rats.

In this figure it is evident that the administration of peptide F19-6, corresponding to the original epitope of the *hHsp60,* and their variant APL F19-7, induced a significant reduction of the clinical signs of the Arthritis in the animals treated with both peptides. It was obtained statistically significant differences among the groups treated with the peptides F19-6 and F19-7 and the group taken as positive control of the illness (P <0.05).

The results obtained in this assay corroborate that peptides F19-6 and F19-7 exert a protective effect in treated animals, since it was possible to observe an almost complete remission of the illness in the rats.

### Example 8: mRNA isolation.

The mononuclear cells isolated from spleens of rats, were incubated approximately during 20 hours. Later, supernatants were discarded and 1mL of the reagent: TRI REAGENT TM (SIGMA, USA) was added to each well and it was left during 5 minutes at room temperature. Later on, the isolation of the mRNA was carrying out following the protocol recommended by the suppliers.

### Example 9: Reverse transcription of RNA.

For obtaining the complementary DNA the system Gene Amp RNA PCR Kit (Perkin - Elmmer, USA) was used. 1 mg of mRNA corresponding to each sample and the protocol was used and the protocol was continued as recommended the suppliers. The reaction was incubated in a thermocycler (Minicycler, MJ Research, USA), according to the following outline: a first cycle of 1 hour at 42°C, subsequently 5 minutes at 95°C and 5 minutes at 4°C.

### Example 10: Polymerase Chain Reaction.

The Polymerase Chain Reaction (PCR) was carried out for the cytokines: TNFα and IL-10 and the constituent gene that codes for the enzyme GAP-DH (glyceraldehyde-3-phosphate dehydrogenase, abbreviated GAP-DH).

For each reaction 10 pmol of each primer was used, 5 mL of the DNA mold, 0.25 mL of the DNA Thermostable Polymerase (Taq-Pol) (Perkin - Elmmer, USA) in the buffer recommended by the suppliers (KCI 50Mm, Tris-HCl 10mM, pH 9.0, Triton X-100 to 0.1 % and MgCl2 1.5 mm) in a final volume of 25 µL.

The reaction for the GAP-DH was incubated in a thermocycler (Minicycler, MJ Research, U.S) according to the following program: a first cycle of 3 minutes at 94°C (denaturating temperature), 1 minute at 94°C, 1 minute at 58°C (hybridization temperature) and 1 minute at 72°C (elongation temperature), subsequently 35 cycles of: 1 minute at 94°C, 1 minute at 58°C and 1 minute at 72°C in each one. Lastly an extension step of 3 minutes at 72°C.

For the cytokines, the previous outline was continued, varying only the hybridization temperature. For the IL-10, 54°C and for the TNFα 64°C.

### Example 11: Analysis of the PCR products by electrophoresis in agarose gels.

The result of the PCR was analyzed by electrophoresis in agarose gel. The images of the electrophoretical running were captured and analyzed with the Molecular Program Analyst (BioRad). The results were expressed as relative levels of mRNA for each cytokine.

### Example 12: Statistical analysis of the relative levels of mRNA for each cytokine.

To determine the differences in the pattern of cytokines (according to the relative levels of mRNA for each one) among groups of treated animals, the tests of Kruskal-Wallis and Student-Newman-Keuls were used. (Statistical package Sigma Stat v 2 1997, GraphPad Software, Inc).

The statistical analyses demonstrated that in the day 15 after the induction of the disease, the peptides significantly diminish the levels of TNFα in comparison with the control group of the illness.

These results are shown in the Figure 2, where GI corresponds to treated animals with F19-6 peptide, GII represents the group of rats treated with the F19-7 peptide, GIII represents the not treated sick group and GIV includes the healthy animals. It was clearly proven that the treatment with peptides caused a decrease in the relative levels of this cytokine responsible in great measure of inducing the inflammatory process in the animals. There were statistically significant differences for the case of the cytokine TNFα among the groups treated with the peptides and in the group used as positive control of the illness. Significant difference was not evidenced among the different groups treated with the peptides neither among them and the group taken as negative control of the illness. This result indicates that the administration of these two peptides caused a decrease in the levels of TNFα, practically until normal levels of this cytokine as in healthy animals.

With the purpose of determining if the peptides used in this study, could stimulate the production of anti-inflammatory cytokines as the IL-10, the relative levels of mRNA corresponding to this cytokine were evaluated.

The IL-10 is involved in the induction of peripheral tolerance through a T cell mediated response. The IL-10 regulates directly the T cells to inhibit the production of IL-2, IL-5 and TNFα.

The Figure 3 shows the quantitative measurement of the levels of expression of the IL-10 in the different groups of treatment, at day 15 after the induction of the illness. In this figure, GI corresponds to treated animals with F19-6 peptide, GII represents the group of rats treated with F19-7 peptide, GIII represents the group of sick animals not treated and GIV includes the healthy animals.

The treatment of the animals with these peptides induces an increment in the relative expression levels of the IL-10. We did not obtain statistically significant differences between groups treated with both peptides, but differences exist between these groups and animals that constitute positive and negative controls. Starting from these results we can infer that the peptides act inducing a change in the functional phenotype of the T cells involved in the pathogenesis of the illness, increasing the levels of production of anti-inflammatory cytokines like the case of IL-10 and diminishing the levels of production of the pro-inflammatory cytokines like TNFα.

### Example 13: Histo-pathological Analysis.

With the objective to corroborate the clinical signs observed in the animals of this animal model and to analyze the capacity of both peptides to revert or to diminish the destructive process generated by the *Mt*, we carried out the pathological analysis of the animal's joints of each group.

The sheets corresponding to the joints were analyzed by the group of Pathology of CIGB.

The joints were fixed in a neutral buffer of formalin at 10% and decalcified. The tissues were dehydrated in an alcohol gradient, absorbed in paraffin and cut in sections of 5µm. Later on, the samples were mounted on glass slides and stained with a hematoxiline-eosine mixture and observed in an optical microscope.

The results of the histological analysis are shown in the table 3.

**Table 3. Summary of the histological analysis of the animal's joints from group I to group IV.**

| **Groups** | **# animal** | **Sacrifice day** | **P** | **EC** | **IM** | **HO** | **G** | **PMN** |
|---|---|---|---|---|---|---|---|---|
| **I (F19-6)** | **1** | | | | **X** | **X** | | **X** |
| | **2** | | | | | | **x/-** | **X** |
| | **3** | **15** | | | | | | |
| **I(F19-6)** | **1** | | | | | | **x/-** | |
| | **2** | | | | | | **x/-** | **X** |
| | **3** | **21** | | | **X** | **X** | **x/-** | **X** |
| **I (F19-6)** | **1** | | | | **X** | **X** | | |
| | **2** | | **X** | **x/-** | **X** | **X** | **x/-** | **X** |
| | **3** | **28** | | | | | | |
| **I (F19-6)** | **1** | | **X** | **x/-** | **X** | **X** | **X** | **X** |
| | **2** | | **X** | | **X** | | **x/-** | |
| | **3** | **35** | **X** | **X** | **X** | **X** | **X** | **X** |
| **II (F19-7)** | **16** | | | | | | | |
| | **17** | | | | | | | |
| | **18** | **15** | | | | | | |
| **II (F19-7)** | **16** | | | | **X** | **X** | **x/-** | **X** |
| | **17** | | | | **X** | **X** | **x/-** | **X** |
| | **18** | **21** | | | **X** | **X** | **x/-** | **X** |
| **II (F19-7)** | **16** | | **X** | **X** | **X** | **X** | **x/-** | **X** |
| | **17** | | **X** | **X** | **X** | **X** | **x/-** | **X** |
| | **18** | **28** | | | | | | |
| **II (F19-7)** | **16** | | | | | | | |
| | **17** | | **X** | **X** | **X** | **X** | **x/-** | **X** |
| | **18** | **35** | | | | | | |
| **III** | **46** | | **X** | **X** | **X** | **X** | **X** | |
| | **47** | **15** | **X** | **X** | | | **X** | |
| | **48** | | **X** | **X** | **X** | **X** | **X** | |
| **III** | **46** | | **X** | **X** | **X** | **X** | **X** | |
| | **47** | | **X** | **X** | **X** | **X** | **X** | |
| | **48** | **21** | **X** | **X** | **X** | **X** | **X** | **X** |
| **III** | **46** | | **X** | **X** | **X** | **X** | **X** | **X** |
| | **47** | | | | **X** | **X** | **X** | **X** |
| | **48** | **28** | **X** | **X** | **X** | **X** | **X** | **X** |
| **III** | **46** | | **X** | **X** | **X** | **X** | **X** | |
| | **47** | | | **X** | **X** | **X** | **X** | |
| | **48** | **35** | **X** | **X** | **X** | **X** | **X** | |

In Table 3 **P:** represents the pannus; **EC:** erosion of the cartilage; **IM:** invasion of the bone marrow; **HO:** hyperplasia of the osteoclasts; **G:** granulematose reaction; **PMN:** polymorphonuclears.

In the animals of the group III including those sacrificed at day 15, was evident the presence of the pannus that cause the erosion of the cartilage and the invasion of the bone marrow. Also, the typical granulomatose reaction of the induction with *Mt* is observed and frequently the hyperplasia of the osteoclasts that are giant cells that have a great destructive power of the bony substance. These histological results reproduce the characteristic signs of the modifications in the joint that occurring in patients with AR and demonstrate that we have developed an appropriate animal model to evaluate the pharmacological candidates.

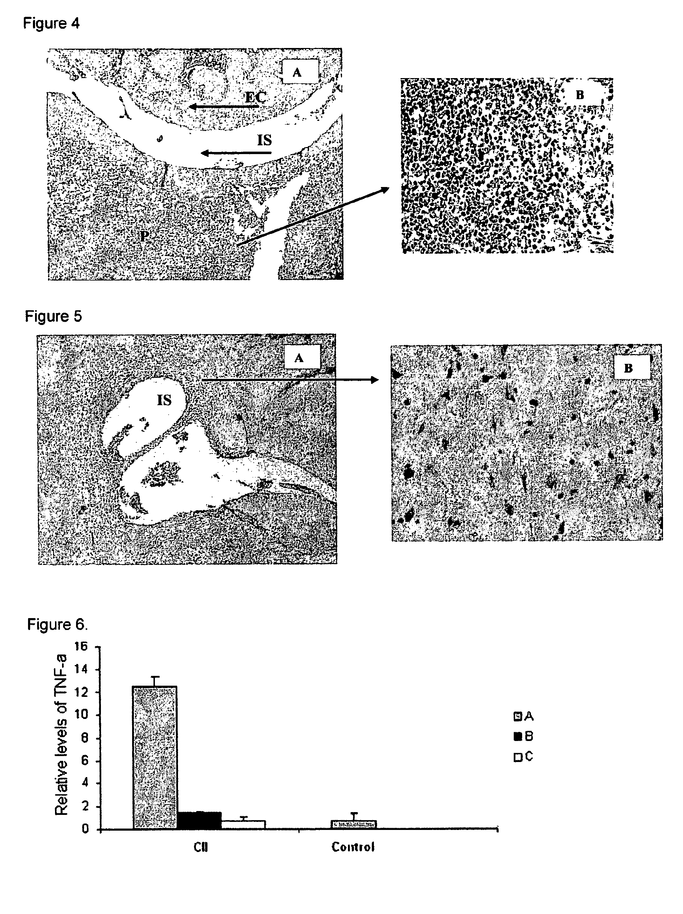

The Figure 4A corresponds to the joint of an animal of the group III. We can observe the presence of the pannus that invades the whole intra-articular space, extending toward the cartilage where its erosion is evidenced, as well as the invasion toward the bone marrow. The Figure 4B shows an amplification of the region corresponding to the bone marrow where the pannus is clearly observed as a granulation tissue rich in phagocytic cells.

In the treatment with F19-7 peptide by intradermal route (group II) as is shown in Figure 5, sacrificed animal at day 15 did not present histological alterations, just the presence of polymorphonuclears and abundant scaring tissues; in contrast with the rest of the animals of the group III, that were characterized to present severe histological signs.

As it is shown in Table 3, the groups of treated animals with the F19-6 and F19-7 peptides didn't show practically histological affectations during the course of the first assay. A bigger protection against to the development of the Arthritis was evidenced in the animals treated with F19-7 peptide. Half of the rats involved in this group of treatment (6 rats) didn't present any histological signs, in correspondence with the group of animals treated with F19-6, where only two rats didn't present histopathological damage. In the groups treated with F19-6 and F19-7 peptides the presence of pannus was not observed at days 15 and 21 after induction of the Arthritis, which indicates that the administration of these peptides caused a retard in the appearance of the cell events characteristic of the inflammatory reaction during the development of the Arthritis. It was significant that in treated animals with F19-7 peptide that were sacrificed at 35 day after induction of the Arthritis, only one rat evidenced the histological affectations characteristic of the development of the illness. These results indicate that the treatment of the animals with this peptide induced, besides a retard in the clinical manifestation of the illness, practically a total remission of the histo-pathological signs of the Arthritis, showing a therapeutic superiority of the F19-7 APL with regard to the peptide containing the original epitope F19-6.

### Example 14. In vitro evaluation of the cytokine pattern induced by the F19-6 and F19-7 peptides, using mononuclear cells from rats where the Arthritis was induced with type II collagen.

In this assay sick rats were used where the Arthritis was induced using bovine type II collagen in IFA. The spleen of each analyzed rat was extracted and the mononuclear cells were isolated.

The mononuclear cells isolated from the spleen were cultured in 1.5 mL of RPMI 1640 in 24 well plates (Costar, USA). The assay was carried out by triplicate, and a total of 3×10⁶ cell/well was cultured during 24 hours with F19-6 and F19-7 peptides at a concentration of 5mg/mL and incubated at 37°C, in humid atmosphere of CO₂ at 5%. The cultivated cells without the peptides were used as negative control.

Later on the mRNA was isolated and the reactions of reverse transcription of the nucleic acid and the PCR were carried out in the same way as described in the previous examples. The analyses of the reaction products were performed by electrophoresis in agarose gels as described in the example 12. To determine the differences in the cytokine pattern according to the relative levels of mRNA for each sample, statistical tests were used as described in example13.

In Figure 6 are shown the levels of TNFα of the cultured cells without peptide and stimulated *in vitro* with F19-6 and F19-7 peptides corresponding to day 21 after the induction of the illness. The letter A represents the levels of TNFα in the cultivated cells without peptide, the letter B represents the values of this cytokine when the cells are stimulated with the F19-6 peptide and the letter C corresponds at the levels of TNFα when the cells are stimulated with the F19-7 peptide. In this figure CII represents the group of animals, where the Arthritis was induced with collagen type II and Control represents the group of healthy animals.

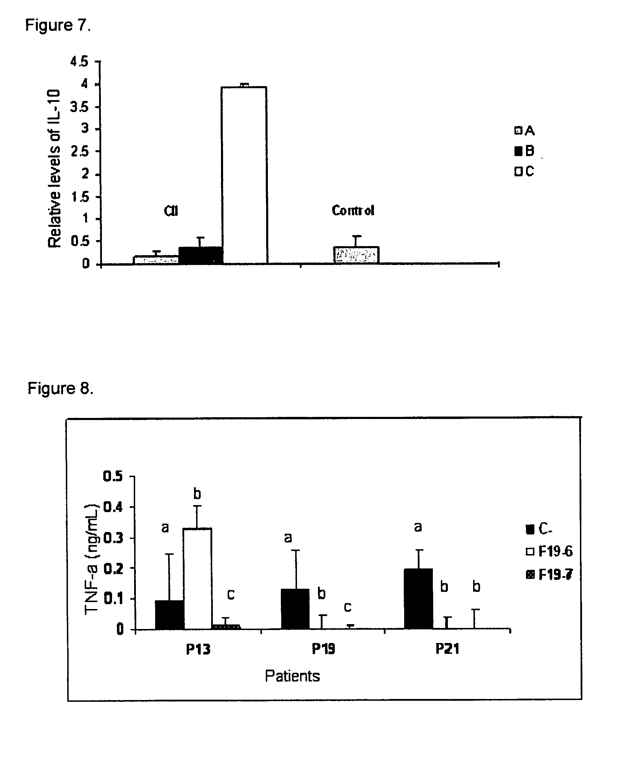

Figure 7 shows the levels of IL-10 of cultivated cells without peptide and stimulated *in vitro* with F19-6 and F19-7 peptides, corresponding to the day 21 after the induction of the illness, the letter A represents the levels of IL-10 in the cultivated cells without peptide, the letter B represents the values of this cytokine when the cells are stimulated with the F19-6 peptide and the letter C corresponds to the levels of IL-10 when the cells are stimulated with F19-7 peptide. In this figure, CII represents the group of animals, where the AR was induced with collagen type II and Control represents the group of healthy animals.

As it is observed in these two figures, F19-6 and F19-7 peptides diminish the levels of TNFα *in vitro,* but in the case of the levels of IL-10, only the F19-7 peptide increases it significantly.

These results indicate that F19-7 peptide is able to modulate the cytokines pattern to a regulating phenotype in the animal model where the Arthritis is induced with collagen. Therefore we can affirm that the immunomodulating action of this peptide derived from the *hHsp60 in vitro* can be extended to animal models of Arthritis, where the induction agent that participates in the development of the illness is not *Mt* or related autoantigens.

These results suggest that the F19-7 peptide can induce tolerance for structurally related antigens, being able to mediate *in vivo* active suppression in the site of the inflammation.

With these results we can suggest that F19-7 peptide induces a protection against arthritis induced by *Mt* or type II collagen in the animals, which is mediated by regulatory T cells that produce IL-10. This effect could extend to other autoantigens present in the joints that progressively contribute to the immunophatogenic process that takes place in the course of RA.

### Example 15: Cytokine measurement in patients with RA induced by F19-6 and F19-7 peptides.

Using blood of patients with RA was carried out assays of measurement of cytokines: TNFα, IFNδ and IL-10. The potentiality of the peptides of the *hHsp60* to increase or to diminish the different cytokines involved in the pathogenesis of the illness was evaluated in this assay.

Starting from 10 mL of blood of each patient that was diluted ½ in PBS 1X, 5 mL from this dilution was added to 3 mL of Ficoll-Paque (Amershan) in centrifugation tubes of 15 mL, which were centrifuged during 30 min. to 1200 rpm. The ring corresponding to mononuclear cells was extracted. Later on, the cells were washed twice with 15 mL of PBS 1X and after each washing, they were centrifuged at 900 rpm. Finally, the pellet of the cell was resuspended in RPMI 1640 containing 10% of bovine fetal serum and supplemented with penicillin (100U/mL), streptomycin (100µg/mL), HEPES 25 mM/L and L-glutamine 2mM (all acquired from Gibco BRL). The mononuclear cells obtained were seeded in number of 10⁶ cells/well in plates of 24 wells (Costar) in a volume of 800 µL. Later on, peptides of the *hHsp60* were added at a concentration range from 0.5 µg/mL up to 100 µg/mL, in order to know the optimimal concentration of each peptide to modulate the cytokines levels. Phytohemagglutinin (PHA) 1% was used as positive control of cellular stimulation, while the RPMI 1640 alone was used as control of the basal cell growth.

The cells were cultured during 24 hours and later on the supernatant of each well was taken, diluted ½, and cytokine concentration was determined by specific kits (Quantikine®, R&D Systems) according to recommendations of suppliers.

The levels of TNFα modulated by F19-6 and F19-7 peptides in mononuclear cells of patients with RA are represented in the Figure 8. In this figure, the letter A-represents cultured cells *in vitro* without peptides; while B corresponds to cells stimulated *in vitro* with F19-6 peptide and C represents cells stimulated *in vitro* with F19-7 peptide. In the figure, when exists statistically significant difference among the levels of cytokines secreted by cells stimulated with peptides with respect to non stimulated cells, a different letter is assigned in bars for levels of the corresponding cytokine.

In the patients represented as P19 and P21, the levels of TNFα diminished significantly by the F19-6 and F19-7 peptides in comparison with the non stimulated cells as it is observed in Figure 8, although in patient P19 the decrease in levels of this cytokine is more significant than with F19-7 peptide.

In the patient represented as P13, the levels of TNFα were increased by the F19-6 peptide, however the levels of this cytokine diminished significantly, when this patient's mononuclear cells are stimulated with the F19-7 peptide. These results evidence that F19-7 peptide, although it was designed to be presented by the MHC from rat, they can diminish the levels of this cytokine, which is largely responsible of the inflammatory process characteristic of RA, thus this peptide is a potential therapeutic candidate for the treatment of this disease.

### Example 16: Cytokines measurement in patients with RA induced by E18-12 and E18-3 peptides.

Assays for cytokines measurement using blood of patients with RA were carried out: TNFα, IFNδ and IL-10. The potentiality of the peptides of the *hHsp60* to increase or to diminish the different cytokines involved in the pathogenesis of the illness was evaluated in these assays.

Ten mL of blood were extracted from each patient, which were diluted ½ in PBS 1X. It was added 5 mL of this dilution to 3 mL of Ficoll-Paque (Amershan) in centrifugation tubes of 15 mL, and centrifuged during 30 min at 1200 rpm. The ring corresponding to mononuclear cells was extracted. Later, the cells were washed twice with 15 mL of PBS 1X and after each washing they were centrifuged at 900 rpm. Finally, the precipitate was resuspended in RPMI 1640 medium containing 10% of bovine fetal serum supplemented with penicillin (100U/mL), streptomycin (100ig/mL), 25 HEPES mM/L and L-glutamine 2mM (all acquired of Gibco BRL).

The mononuclear cells obtained were seeded in a number of 10⁶ cells/well in plates of 24 wells (Costar) in a volume of 800 µL. Afterwards, the peptides of the *hHsp60* were added in a concentration range from 0.5 µg/ml up to 100 µg/ml, in order to know the optimal concentration of each peptide to modulate the cytokine levels. The Phytohemagglutinin (PHA) to 1% it was used as positive control of cell stimulation, while the RPMI 1640 alone was used as control of the basal cell growth.

The cells were cultured during 24 hours and later on, the supernatant of each well was taken, diluted ½, and cytokine concentration was determined by specific kits (Quantikine®, R&D Systems) according to the recommendations of the suppliers.

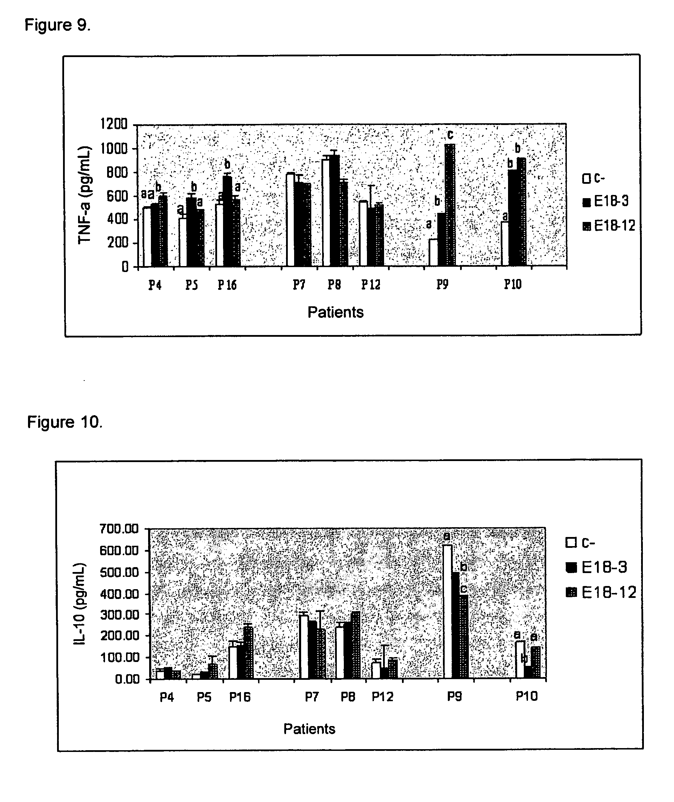

The levels of TNFα modulated by the E18-3 and E18-12 peptides in mononuclear cells of patients with RA are represented in Figure 9. Patients represented by P4, P5 and P16 express the molecule DR 0306, the patients identified as P7, P8 and P12 express the molecule DR 0303, while patient P9 presents a genotype DR0506 and patient P10 has a genotype DR 0204. In this figure, "C-" (white bars) represents the cultured cells *in vitro* without peptides; while "E18-3" (blach bars) corresponds to the cells stimulated in vitro with E18-3 peptide and "E18-12" (crossed bars) represents cells stimulated *in vitro* with E18-12 peptide.

In the figure when exists statistically significant difference among the levels of the cytokines secreted by the cells stimulated with the peptides with respect to non stimulated cells, a different letter is assigned in the bars for levels of the corresponding cytokine.

As it is observed in Figure 9, the induction of the synthesis of TNFα in mononuclear cells of patients, stimulated with E18-12 and E18-3 peptides depends on the HLA type II molecules that these patients express. In patients P4, P5 and P6 that present a genotype DR 0306, a statistically significant increment of the concentration of TNFα was observed, in comparison with the cells not stimulated with peptides.

This same effect takes place in the patient P9 of genotype DR 0506 and the patient P10 DR 0204.

In patients P7, P8 and P12 presenting a genotype DR 0303, any difference in levels of TNFα was not observed, when mononuclear cells are stimulated with our peptides.

The modulation of IL-10 levels for E18-3 and E18-12 peptides are represented in Figure 10, where "C-" (white bars) represents cultured cells *in vitro* without peptides; while "E18-3" (blach bars) corresponds to cells stimulated in vitro with the E18-3 peptide and "E18-12" (crossed bars) represents cells stimulated *in vitro* with E18-12 peptide.

In the case of IL-10, as it is observed in Figure 10, there is a significant decrease of this cytokine in the patients P9 and P10, but in the rest of the patients were not significant changes.

From these observations we can infer that E18-12 and E18-3 peptides induce a TH1 phenotype in peripheral mononuclear cells.

A therapeutic variant of original peptides: E18-12 or E18-3 could be their administration by oral route, so they will induce mechanisms of tolerance that contributed significantly to decrease the inflammation.

### Example 17: Cytokine measurement in patients with RA induced by derived type APL peptides.

From previous results, we decided to modify these original peptides corresponding to epitopes of T cells, at the sites of contact with HLA molecule, using Bioinformatic tools, so that the modified type APL peptides would be able to change the pattern of cytokines to a regulating phenotype. In addition, modifications made in each of these peptides fulfill with peptide binding motifs reported for most of the HLA molecules expressed by the patients that theoretically will favor the interaction of each of them with different HLA molecules.

Assays for measurement of the cytokines: TNFα and IL-10, were carry out using blood of patients with RA. The potentiality of derived type APL peptides from the *hHsp60* to increase or to diminish the different cytokines involved in the pathogenesis of the disease was evaluated in these tests. In addition, the original peptides were included in this evaluation (E18-3, E18-12) as experimental controls.

The APL peptides evaluated in these tests were the following:
MGPKGRTVIILQSWGSPKVTK (SEQ. ID. NO: 5)
MGPKGRTVII**M**QSWGSPKVTK (SEQ. ID. NO: 6)
MGPKGRTVII**A**QSWGSPKVTK (SEQ. ID. NO: 7)
MGPKGRTVIIEQSLGSPKVTK (SEQ. ID. NO: 8)
MGPKGRTVIIEQS**M**GSPKVTK (SEQ. ID. NO: 9)
MGPKGRTVIILQSLGSPKVTK (SEQ. ID. NO: 10)
MGPKGRTVIILQS**M**GSPKVTK (SEQ. ID. NO: 11)
MGPKLRTVIIEQSWGSPKVTK (SEQ. ID. NO: 12)
MGPKLRTVIILQS**M**GSPKVTK (SEQ. ID. NO: 13)
SIDLKDKLKNIGAKLVQDVANNTNEEA (SEQ. ID. NO: 14)
SIDLKDKYKNIGAKLSQDVANNTNEEA (SEQ. ID. NO: 15)
SIDLKDKLKNIGAKLSQDVANNTNEEA (SEQ. ID. NO: 16)
SIDLKDKYKNAGAKLVQDVANNTNEEA (SEQ. ID. NO: 17)
SIDLKDKYKNIGAKLVQLVANNTNEEA (SEQ. ID. NO: 18)
SIDLKDKYKNIGAKLVQAVANNTNEEA (SEQ. ID. NO: 19)
SIDLKDKYKNIGAKLVQDVANNANEEA (SEQ. ID. NO: 20)
SIDLKDKYKNIGAKLVQLVANNANEEA (SEQ. ID. NO: 21)

Ten mL of blood were extracted from each patient and diluted ½ in PBS 1X. It was added 5 mL of this dilution to 3 mL of Ficoll-Paque (Amershan) in centrifugation tubes of 15 mL, and centrifuged during 30 min at 1200 rpm. The ring corresponding to mononuclear cells was extracted. Later, the cells were washed twice with 15 mL of PBS 1X and after each washing they were centrifuged at 900 rpm. Finally, the precipitate was resuspended in RPMI 1640 medium containing 10% of bovine fetal serum supplemented with penicillin (100U/mL), streptomycin (100µg/mL), 25 HEPES mM/L and L-glutamine 2mM (all acquired of Gibco BRL).

Obtained mononuclear cells were cultured in number of 10⁶ cells / well in plates of 24 wells (Costar) in a volume of 800 µL. Cells were stimulated with peptides [E18-3, E18-12, E18-3APL1 (SEQ. ID. NO: 18), E18-3APL2 (SEQ. ID. NO: 21), E18-12APL1 (SEQ. ID. NO: 5), E18-12APL3 (SEQ. ID. NO: 12)] at a concentration of 40 µg/mL and by triplicate. RPMI 1640 was used as control of the basal cellular growth.

Cells were cultured during 24 hours and later supernatants of each well were taken, diluted ½ and the concentration of cytokines were determined by specific kits (Quantikine®, R&D Systems) according to the recommendations of the suppliers.

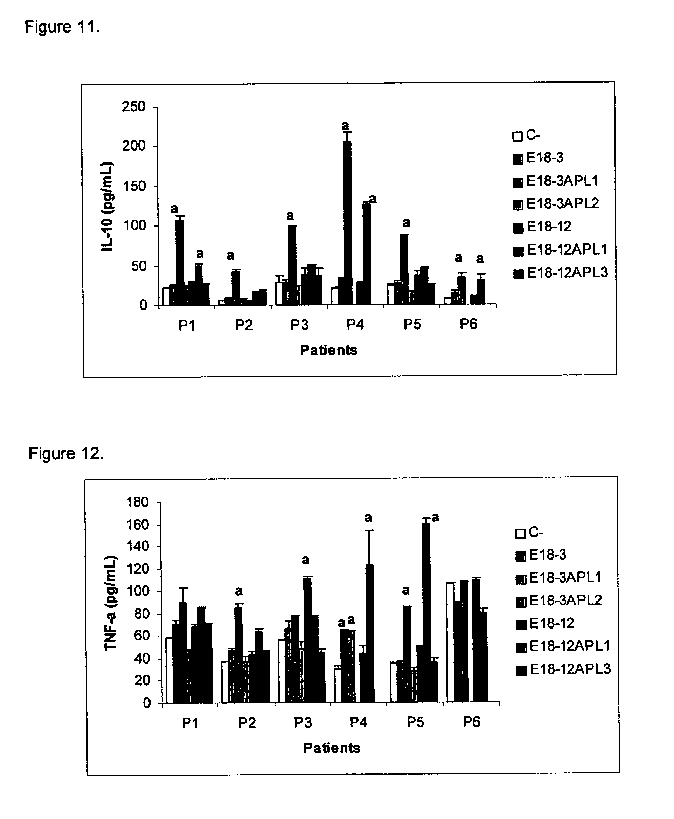

In figure 11 is shown the levels of IL-10 induced by original peptides (E18-12 and E18-3) and a peptide panel of APLs: E18-3APL1 (SEQ.ID. NO: 18); E18-3APL2 (SEQ. ID. NO: 21); E18-12APL1 (SEQ. ID. NO: 5); E18-12APL3 (SEQ. ID. NO: 12).

In this figure, the letters indicate statistically significant differences (we only refer statistical significance between the different peptides and control cells in each patient individually). In addition, cells of patients P4 and P6 were not stimulated by peptides E18-3APL2 nor E18-12APL3. It is evident that, in all patients, peptide E18-3APL1 increases the levels of IL-10 very significantly with respect to the non stimulated cells. The increase induced by this peptide in some of the patients, as P2 and P4, was about 9 times higher than the amount produced by the cells without stimulation, and in patients as P1, P3 and P5 about 5 times superior. Also, similar results were obtained with E18-12APL1 due to a significant increase in levels of IL-10 in patients P1, P4 and P6. On the other hand, neither the original peptides nor peptides E18-3APL2 and E18-12APL3 induced an increase in the levels of IL-10 in comparison with the cells that represent the negative control.

In figure 12 is shown the levels of TNFα induced by different peptides in the same group of patients. In this figure, the letters indicate statistically significant differences (we only refer statistical significance between different peptides and control cells in each patient individually). In addition, cells of patients P4 and P6 were not stimulated by peptides E18-3APL2 nor E18-12APL3). As it can be appraised, all the patients do not respond with the same intensity to the stimulus with different peptides, although it is notorious that the response was quite homogenous. On the other hand, the peptide that increased more the production of TNFα was the E18-12APL1, mainly in patients P4 and P5, whereas patients: P1, P2 and P3 showed a slight increase with respect to the negative control. Also, peptide E18-3APL1 had a similar behavior in most of the patients. In the case of peptide E18-3APL1 the cells of the patients P2, P4 and P5, showed the major increase of the levels of TNFα, although was less marked. In this test, we obtained similar results to previous evaluations with the original peptides. The previous results indicate that neither peptide nor its APL induce a decrease of TNFα expression in mononuclear cells of the patients.

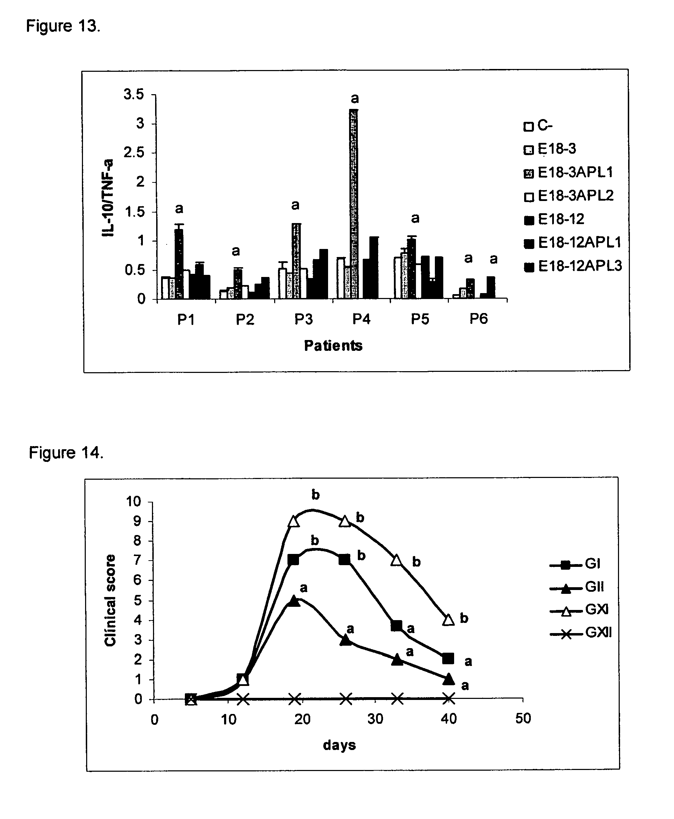

These results could be contradictory, since such APLs that increase the production of IL-10 promote an increase in the levels of TNFα. In figure 13 is shown ratio of the levels of IL-10 and TNFα induced by original peptides and their APLs. In this figure, the letters indicate statistically significant differences (we only refer statistical significance between different peptides and control cells in each patient individually). In addition, cells of patients P4 and P6 were not stimulated by peptides E18-3APL2 nor E18-12APL3). It is evident the fact that the increase of IL-10 with respect to TNFα levels was considerably higher, because the cells stimulated with the E18-3APL1 showed an index higher than 1 in four out of six patients, which indicates that IL-10 levels exceeded to those of TNFα. In addition, in all patients, IL-10 levels were considerably higher in cells stimulated with peptide E18-3APL1 with respect to cells that constituted the negative control, showing statistically significant differences. In the case of the E18-12APL1 the differences in comparison to the negative control were not so remarkable. So, although E18-3APL1 increases the levels of TNFα, the net predominant effect is the increase in the levels of IL-10, which is translated in a polarization of the response in favor of immunosuppressor cytokines.

The results of the study are extremely promissory, because it suggested that these two APLs could be administered to patients and could induce a subpopulation of regulatory T cells secretory of IL-10 that mediate an active suppression at systemic level. Another partial conclusion that we can arrive by means of the analysis of these results, is that these APLs practically display a homogenous behavior in all the studied patients, independently of the genotype that they have. These results are extremely encouraging, because it would allow that the administration of these APL had the same effect in all the patients, in spite of the existing differences with respect to the type of HLA-II molecules that they have.

From our experimental results, the fundamental advantage of the present invention is the use of pharmaceutical preparations containing immunomodulating peptides of the human stress protein *Hsp60,* corresponding to T cell epitopes of or variants type APL of these peptides, that can be effectively used in the treatment of patients with RA, inducing a specific immune response directed to silence the clones of involved autorreactive T cells in the immunopathogenic mechanisms of this disease.

The treatment we propose in this invention is reasonable and can be extended to a large number of patients and also it can be used in combination with current therapies for this disease.

### Example 18: Evaluation of the therapeutic effect of derived type APL peptides, in an animal model where arthritis is induced with Mt.

The animals were separated in twelve groups of eight rats each one. The disease was induced in eleven animal groups (Grupo I- Grupo XI), ten of them were treated with peptides and one stayed as control of the induction of the disease. Animal groups that were treated with peptides, received 50 µg of peptide by rat in a volume of 100 µL of PBS, days 12, 14, 16, 18, 20, 22, 24, 26 after induction of the disease with *Mt.*

The separated animals in twelve groups were treated with the following form:
➢ Group I: received peptide E18-12 (SEQ. ID. NO: 1)
➢ Group II: received peptide corresponding to: SEQ. ID. NO: 5
➢ Group III: received peptide corresponding to: SEQ. ID. NO: 8
➢ Group IV: received peptide corresponding to: SEQ. ID. NO: 12
➢ Group V: received peptide corresponding to: SEQ. ID. NO: 13
➢ Group VI: received peptide E18-3 (SEQ. ID. NO: 2)
➢ Group VII: received peptide corresponding to: SEQ. ID. NO: 14
➢ Group VIII: received peptide corresponding to: SEQ. ID. NO: 15
➢ Group IX: received peptide corresponding to: SEQ. ID. NO: 16
➢ Group X: received peptide corresponding to: SEQ. ID. NO: 17
➢ Group XI: do not received peptide (control of disease induction)
➢ Group XII: the disease was not induced or administered any peptide (negative control).

Days 21 and 35 after the induction, several animals from each group were sacrificed, spleens were extracted and sample was taken from joints for histopathological analysis.

In the groups of animals treated with peptides type APL, the clinical improvement was significant in comparison with the non treated animal group and with the groups treated with E18-12 and E18-3. These results were corroborated by histopathological analysis and by measurement of cytokines, demonstrating in all cases a superiority of derived type APL peptides upon original peptides.

In figure 14 we show the graphic of evolution of the disease in four animal groups pertaining to this animal model. In this case, the groups are: group I (animals treated with E18-12), group II (animals treated with E18-12APL1 SEQ. ID. NO: 5), group XI (control of the disease induction) and group XII (healthy animals). In this graph, different letters indicate significant differences (p<0.001) (it refers to the statistical significant between all groups in measured specific days.

It is possible to appreciate in this graph, that from day 12 after the induction of the disease, the animals of groups I, II and XI began to show the characteristic clinical signs of RA. Many animals also presented extra-articular manifestations like nodules in the ears, in the tail and in the extremities. The most severe manifestations of the RA were observed around day 20 for the three ill animal groups. In the case of animals treated with E18-12, in day 21 we could not appreciate significant differences with respect to the control of the disease induction group. Nevertheless, around day 35 after the induction of the disease, clinical signs in animals of this group were significantly inferior to those of group XI (p<0.001). These results indicate that protective effect of the E18-12 peptide is shown around day 35. In this sense, it would be recommendable to administer this peptide in earlier phases of the disease in order to obtain better results at day 21. However, in the case of animals of groups II the clinical signs of inflammation were evidently smaller in comparison with groups I and XI (p<0.001), which indicates that this peptide exerts a potent protective effect in the animals where the disease was induced.

## Claims

1. Peptide of the human heat shock protein of 60 kDa that constitute epitopes for T cells, for use in inducing mechanisms of peripheral tolerance, in particular anergy induction or mediated by clones of regulatory T cells in patients with Rheumatoid Arthritis wherein said peptide is **characterized by** the sequence:
E18-3 SIDLKDKYKNIGAKLVQDVANNTNEEAG (SEQ. ID. NO: 2).

2. Peptide, which is **characterized** to be an Altered Peptide Ligand (APL) peptide derived from the E18-3 peptide (SEQ.ID. NO: 2) of claim 1, modified at the contact sites with the human major histocompatibility complex (MHC) molecules, wherein the amino acids sequence: is substituted in
position 1 for L,I,V,M,Y,W,F, or A
position 2 for A,D,E,F,G,H,I,K,L,M,N,Q,R,S,T,V,W, or Y
position 3 for A,F,I,K,L,M,P,R,S,T,V,W, or Y
position 4 for L,I,V,M,Y,W,F, or A
position 5 for A,D,E,F,G,H,I,K,L,M,N,Q,R,S,T,V,W, or Y
position 6 for A,D,E,F,G,H,I,K,L,M,N,Q,R,S,T,V,W, or Y
position 7 for A,F,I,K,L,M,P,R,S,T,V,W, or Y
position 8 for A,D,E,F,G,H,I,K,L,M,N,Q,R,S,T,V,W, or Y

3. Peptide according to claim 2, having an amino acid sequence selected from the group consisting of: SEQ. ID. NO: 14, SEQ. ID. NO: 15, SEQ. ID. NO: 16, SEQ. ID. NO: 17, SEQ. ID. NO: 18, SEQ. ID. NO: 19, SEQ. ID. NO: 20 and SEQ. ID. NO: 21.

4. A pharmaceutical composition comprising one or more peptides of any one of claims 1 to 3 and a pharmaceutically acceptable vehicle.

5. Pharmaceutical composition according to claim 4, for use in treating Rheumatoid Arthritis.

6. Use of an effective amount of peptides according to any of claims 1 to 3 or a pharmaceutical composition according to claim 4 or 5 for the preparation of a medicament for treating a human subject suffering from Rheumatoid Arthritis.

## Patentansprüche

1. Peptid des menschlichen Heatshock-Proteins mit 60 kDa, das Epitope für T-Zellen darstellt, zur Verwendung beim Induzieren von Mechanismen der peripheren Toleranz, insbesondere Anergieinduktion oder durch Klone regulatorischer T-Zellen bei Patienten mit rheumatoider Arthritis vermittelt, wobei das Peptid durch die Sequenz:
E18-3 SIDLKDKYKNIGAKLVQDVANNTNEEAG (SEQ ID NO: 2)
**gekennzeichnet** ist.

2. Peptid, das als ein verändertes Peptidliganden ("Altered Peptide Ligand", APL) - Peptid, abgeleitet von dem Peptid E18-3 (SEQ ID NO: 2) nach Anspruch 1, **gekennzeichnet** ist, an den Kontaktstellen mit den menschlichen Haupthistokompatibilitätskomplex (MHC) - Molekülen modifiziert, wobei die Aminosäuresequenz in Position 1 durch L, I, V, M, Y, W, F oder A
in Position 2 durch A, D, E, F, G, H, I, K, L, M, N, Q, R, S, T, V, W oder Y
in Position 3 durch A, F, I, K, L, M, P, R, S, T, V, W oder Y
in Position 4 durch L, I, V, M, Y, W, F oder A
in Position 5 durch A, D, E, F, G, H, I, K, L, M, N, Q, R, S, T, V, W oder Y
in Position 6 durch A, D, E, F, G, H, I, K, L, M, N, Q, R, S, T, V, W oder Y
in Position 7 durch A, F, I, K, L, M, P, R, S, T, V, W oder Y
in Position 8 durch A, D, E, F, G, H, I, K, L, M, N, Q, R, S, T, V, W oder Y substituiert ist.

3. Peptid gemäß Anspruch 2 mit einer Aminosäuresequenz, ausgewählt aus der Gruppe, bestehend aus SEQ ID NO: 14, SEQ ID NO: 15, SEQ ID NO: 16, SEQ ID NO: 17, SEQ ID NO: 18, SEQ ID NO: 19, SEQ ID NO: 20 und SEQ ID NO: 21.

4. Pharmazeutische Zusammensetzung, umfassend ein oder mehrere Peptide nach einem der Ansprüche 1 bis 3 und einen pharmazeutisch verträglichen Träger.

5. Pharmazeutische Zusammensetzung gemäß Anspruch 4 zur Verwendung beim Behandeln von rheumatoider Arthritis.

6. Verwendung einer wirksamen Menge Peptide gemäß einem der Ansprüche 1 bis 3 oder einer pharmazeutischen Zusammensetzung gemäß Anspruch 4 oder 5 zur Herstellung eines Medikaments zum Behandeln eines menschlichen, an rheumatoider Arthritis leidenden Probanden.

## Revendications

1. Peptide de la protéine de choc thermique humaine de 60 kDa qui constitue des épitopes pour les lymphocytes T, destiné à une utilisation dans les mécanismes d'induction de tolérance périphérique, en particulier l'induction d'anergie ou médiée par des clones de lymphocytes T régulateurs chez des patients atteints d'arthrite rhumatoïde, dans lequel ledit peptide est **caractérisé par** la séquence :
E18-3 SIDLKDKYKNIGAKLVQDVANNTNEEAG (SEQ ID NO: 2).

2. Peptide, qui est **caractérisé** comme étant un ligand peptidique altéré (APL), peptide dérivé du peptide E18-3 (SEQ ID NO: 2) selon la revendication 1, modifié aux sites de contact avec les molécules du complexe majeur d'histocompatibilité (CMH) humain, où la séquence d'acides aminés : est remplacée dans
la position 1 par L, I, V, M, Y, W, F ou A
la position 2 par A, D, E, F, G, H, I, K, L, M, N, Q, R, S, T, V, W ou Y
la position 3 par A, F, I, K, L, M, P, R, S, T, V, W ou Y
la position 4 par L, I, V, M, Y, W, F ou A
la position 5 par A, D, E, F, G, H, I, K, L, M, N, Q, R, S, T, V, W ou Y
la position 6 par A, D, E, F, G, H, I, K, L, M, N, Q, R, S, T, V, W ou Y
la position 7 par A, F, I, K, L, M, P, R, S, T, V, W ou Y
la position 8 par A, D, E, F, G, H, I, K, L, M, N, Q, R, S, T, V, W ou Y.

3. Peptide selon la revendication 2, ayant une séquence d'acides aminés choisie parmi le groupe consistant en :
SEQ ID NO: 14, SEQ ID NO: 15, SEQ ID NO: 16, SEQ ID NO: 17, SEQ ID NO: 18, SEQ ID NO: 19, SEQ ID NO: 20 et SEQ ID NO: 21.

4. Composition pharmaceutique comprenant un ou plusieurs peptides selon l'une quelconque des revendications 1 à 3 et un véhicule pharmaceutiquement acceptable.

5. Composition pharmaceutique selon la revendication 4, destinée à une utilisation dans le traitement de l'arthrite rhumatoïde.

6. Utilisation d'une quantité efficace de peptides selon l'une quelconque des revendications 1 à 3 ou d'une compositions pharmaceutique selon la revendication 4 ou 5 pour la préparation d'un médicament pour traiter un sujet humain souffrant d'arthrite rhumatoïde.


### List of Sequences

<110> Centro de Ingenieria Genética y Biotecnologia
<120> PEPTIDES AND THEIR DERIVED TYPE APL OF THE HSP60 AND PHARMACEUTICAL COMPOSITIONS.
<130> pepMC
<140>
   <141>
<160> 21
<170> PatentIn Ver. 2.1
<210> 1
   <211> 21
   <212> PRT
   <213> Artificial Sequence
<220>
   <223> Description of the Artificial Sequence: peptide
<400> 1
   Met Gly Pro Lys Gly Arg Thr Val Ile Ile Glu Gln Ser Trp Gly Ser
   1 5 10 15
   Pro Lys Val Thr Lys
   20
<210> 2
   <211> 28
   <212> PRT
   <213> Artificial Sequence
<220>
   <223> Description of the Artificial Sequence: peptide
<400> 2
   Ser Ile Asp Leu Lys Asp Lys Tyr Lys Asn Ile Gly Ala Lys Leu Val
   1 5 10 15
   Gln Asp Val Ala Asn Asn Thr Asn Glu Glu Ala Gly
   20 25
<210> 3
   <211> 16
   <212> PRT
   <213> Artificial Sequence
<220>
   <223> Description of the Artificial Sequence: peptide
<400> 3
   Ile Ile Asp Pro Thr Lys Val Val Arg Thr Ala Leu Leu Asp Ala Ala
   1 5 10 15
<210> 4
   <211> 16
   <212> PRT
   <213> Artificial Sequence
<220>
   <223> Description of the Artificial Sequence: peptide
<400> 4
   Ile Ile Asp Pro Thr His Val Val Arg Thr Glu Leu Leu Asp Ala Ala
   1 5 10 15
<210> 5
   <211> 21
   <212> PRT
   <213> Artificial Sequence
<220>
   <223> Description of the Artificial Sequence: peptide
<400> 5
   Met Gly Pro Lys Gly Arg Thr Val Ile Ile Leu Gln Ser Trp Gly Ser
    1               5                  10                  15
   Pro Lys Val Thr Lys
                20
<210> 6
   <211> 21
   <212> PRT
   <213> Artificial Sequence
<220>
   <223> Description of the Artificial Sequence: peptide
<400> 6
   Met Gly Pro Lys Gly Arg Thr Val Ile Ile Met Gln Ser Trp Gly Ser
    1               5                  10                  15
   Pro Lys Val Thr Lys
                20
<210> 7
   <211> 21
   <212> PRT
   <213> Artificial Sequence
<220>
   <223> Description of the Artificial Sequence: peptide
<400> 7
<210> 8
   <211> 21
   <212> PRT
   <213> Artificial Sequence
<220>
   <223> Description of the Artificial Sequence: peptide
<400> 8
   Met Gly Pro Lys Gly Arg Thr Val Ile Ile Glu Gln Ser Leu Gly Ser
   1 5 10 15
   Pro Lys Val Thr Lys
   20
<210> 9
   <211> 21
   <212> PRT
   <213> Artificial Sequence
<220>
   <223> Description of the Artificial Sequence: peptide
<400> 9
   Met Gly Pro Lys Gly Arg Thr Val Ile Ile Glu Gln Ser Met Gly Ser
   1 5 10 15
   Pro Lys Val Thr Lys
   20
<210> 10
   <211> 21
   <212> PRT
   <213> Artificial Sequence
<220>
   <223> Description of the Artificial Sequence: peptide
<400> 10
   Met Gly Pro Lys Gly Arg Thr Val Ile Ile Leu Gln Ser Leu Gly Ser
   1 5 10 15
   Pro Lys Val Thr Lys
   20
<210> 11
   <211> 21
   <212> PRT
   <213> Artificial Sequence
<220>
   <223> Description of the Artificial Sequence: peptide
<400> 11
   Met Gly Pro Lys Gly Arg Thr Val Ile Ile Leu Gln Ser Met Gly Ser
   1 5 10 15
   Pro Lys Val Thr Lys
   20
<210> 12
   <211> 21
   <212> PRT
   <213> Artificial Sequence
<220>
   <223> Description of the Artificial Sequence: peptide
<400> 12
   Met Gly Pro Lys Leu Arg Thr Val Ile Ile Glu Gln Ser Trp Gly Ser
    1               5                  10                  15
   Pro Lys Val Thr Lys
               20
<210> 13
   <211> 21
   <212> PRT
   <213> Artificial Sequence
<220>
   <223> Description of the Artificial Sequence: peptide
<400> 13
   Met Gly Pro Lys Leu Arg Thr Val Ile Ile Leu Gln Ser Met Gly Ser
    1               5                  10                  15
   Pro Lys Val Thr Lys
               20
<210> 14
   <211> 27
   <212> PRT
   <213> Artificial Sequence
<220>
   <223> Description of the Artificial Sequence: peptide
<400> 14
   Ser Ile Asp Leu Lys Asp Lys Leu Lys Asn Ile Gly Ala Lys Leu Val
    1               5                  10                  15
   Gln Asp Val Ala Asn Asn Thr Asn Glu Glu Ala
               20                  25
<210> 15
   <211> 27
   <212> PRT
   <213> Artificial Sequence
<220>
   <223> Description of the Artificial Sequence: peptide
<400> 15
   Ser Ile Asp Leu Lys Asp Lys Tyr Lys Asn Ile Gly Ala Lys Leu Ser
   1 5 10 15
   Gln Asp Val Ala Asn Asn Thr Asn Glu Glu Ala
   20 25
<210> 16
   <211> 27
   <212> PRT
   <213> Artificial Sequence
<220>
   <223> Description of the Artificial Sequence: peptide
<400> 16
   Ser Ile Asp Leu Lys Asp Lys Leu Lys Asn Ile Gly Ala Lys Leu Ser
   1 5 10 15
   Gln Asp Val Ala Asn Asn Thr Asn Glu Glu Ala
   20 25
<210> 17
   <211> 27
   <212> PRT
   <213> Artificial Sequence
<220>
   <223> Description of the Artificial Sequence: peptide
<400> 17
   Ser Ile Asp Leu Lys Asp Lys Tyr Lys Asn Ala Gly Ala Lys Leu Val
   1 5 10 15
   Gln Asp Val Ala Asn Asn Thr Asn Glu Glu Ala
   20 25
<210> 18
   <211> 27
   <212> PRT
   <213> Artificial Sequence
<220>
   <223> Description of the Artificial Sequence: peptide
<400> 18
   Ser Ile Asp Leu Lys Asp Lys Tyr Lys Asn Ile Gly Ala Lys Leu Val
   1 5 10 15
   Gln Leu Val Ala Asn Asn Thr Asn Glu Glu Ala
   20 25
<210> 19
   <211> 27
   <212> PRT
   <213> Artificial Sequence
<220>
   <223> Description of the Artificial Sequence: peptide
<400> 19
   Ser Ile Asp Leu Lys Asp Lys Tyr Lys Asn Ile Gly Ala Lys Leu Val
   1 5 10 15
   Gln Ala Val Ala Asn Asn Thr Asn Glu Glu Ala
   20 25
<210> 20
   <211> 27
   <212> PRT
   <213> Artificial Sequence
<220>
   <223> Description of the Artificial Sequence: peptide
<400> 20
   Ser Ile Asp Leu Lys Asp Lys Tyr Lys Asn Ile Gly Ala Lys Leu Val
   1 5 10 15
   Gln Asp Val Ala Asn Asn Ala Asn Glu Glu Ala
   20 25
<210> 21
   <211> 27
   <212> PRT
   <213> Artificial Sequence
<220>
   <223> Description of the Artificial Sequence: peptide
<400> 21
   Ser Ile Asp Leu Lys Asp Lys Tyr Lys Asn Ile Gly Ala Lys Leu Val
   1 5 10 15
   Gln Leu Val Ala Asn Asn Ala Asn Glu Glu Ala
   20 25